# EUROPEAN PATENT SPECIFICATION

(11) **EP 1 024 798 B1**
(45) Date of publication and mention of the grant of the patent: **09.10.2002**
(21) Application number: 98937771.8
(22) Date of filing: 30.07.1998
(51) Int. Cl.: A61K 31/00, A61P 43/00

(54) **NEOMYCIN/DISACCHARIDE WATER SOLUTION FOR ENTEROCLYSIS IN HEPATIC ENCEPHALOPATHY**
NEOMYCIN/DISACCHARID WÄSSERIGE LÖSUNG ZUR BEHANDLUNG VON ENTEROCLYSIS BEI HEPATISCHER ENZEPHALOPATHIE
SOLUTION AQUEUSE D'ENTEROCLYSE POUR LE TRAITEMENT DE L'ENCEPHALOPATHIE HEPATIQUE

(30) Priority: 01.08.1997 IT SA970016
(43) Date of publication of application: 09.08.2000
(60) Divisional application: 01129787.6
(73) Proprietor: Labruzzo, Carla, 20148 Milano (IT); Vicidomini, Francesco, 84014 Nocera Inferiore (IT)
(72) Inventor: VICIDOMINI, Francesco, I-84014 Nocera Inferiore (IT)
(74) Representative: Dragotti, Gianfranco
(86) International application number: IT9800220
(87) International publication number: WO99006033

(56) References cited:
- US-A- 3 558 774
- BLANC P. ET AL: "Association lactulose-néomycine versus placebo dans le traitement de l'encéphalopathie hépatique aigue" GASTROENTEROL CLIN BIOL 1994, 18 (1) P1063-8, XP002090631 Paris
- MORGAN M.Y.: "The treatment of chronic hepatic encephalopathy" HEPATO-GASTROENTEROLOGY, 1991, 38/5 (377-387), XP002090635 Germany
- WEBER F. L. JR: "Effects of lactulose on nitrogen metabolism" SCANDINAVIAN JOURNAL OF GASTROENTEROLOGY, 1997, 32/SUP 222 (83-87), XP002090637
- PATENT ABSTRACTS OF JAPAN vol. 007, no. 028 (C-149), 4 February 1983 & JP 57 185214 A (KYOWA HAKKO KOGYO KK), 15 November 1982
- SCEVOLA D. ET AL: "Nonabsorbable disaccharides plus neomycin in hepatic encephalopathy: do they enhance each other?" HEPATOLOGY, vol. 12, no. 2, August 1990, pages 368-370, XP002090931 & SCEVOLA D. ET AL: CLINICA TERAPEUTICA, vol. 129, 1989, pages 105-111,
- CIPOLLINI F. ET AL: "La terapia dell'encefalopatia epatica" G CLIN MED, vol. 63, 1982, pages 753-767, XP002091124
- FINGEROTE R.J. ET AL: "Fulminant hepatic failure" AM. J. GASTROENTEROL., 1993, 88/7 (1000-1010), XP002090639 USA

## Description

The subject of the present invention is an aqueous enteroclysis solution containing, as active ingredients, at least one non-fermentable disaccharide, preferably lactulose or lactitol, in combination with neomycin, for use in the treatment of hepatic encephalopathy.

Hepatic encephalopathy is a neuropsychic syndrome which may occur in patients suffering from hepatic insufficiency, in cases of both acute and chronic liver disease. It may take different shapes which however present common clinical characteristics as mind state alteration, neurologic deficit, hepatic parenchyma degeneration.

Porto-systemic encephalopathy (PSE), which occurs almost exclusively in patients suffering from advanced cirrhosis (viral, alcoholic, etc.) with portal hypertension, is the most typical form of hepatic encephalopathy; its pathogenesis is determined by the so-called "porto-systemic shunt" that is, by a diversion of the blood flow on the basis of which the collateral veins admit the blood from portal vein directly into the systemic circulation, that is, without passing through the hepatic filter. Potentially toxic substances, for example, such as ammonia or other nitrogenous toxins which are normally removed by the liver may therefore reach the brain cells, replace the normal neurotransmitters, and thus generate encephalopathy.

Amongst the factors which promote the occurrence of PSE, hyperazotemia, the use of sedatives, tranquillizers or analgesics, episodes of digestive haemorrhage, metabolic alkalosis conditions, excessive administration of proteins, infections and constipation may be mentioned.

The course of PSE and, more generally, of hepatic encephalopathy, is normally divided into four stages which, in turn, are characterized by different alterations in the state of consciousness:
1. first or prodromic stage;
2. second or imminent coma stage;
3. third or vigil coma stage;
4. fourth or declared coma stage, characterized by a state of complete unconsciousness of the patient without any response to external stimuli.

The current treatment for PSE is normally divided into two distinct stages which provide:
- on the one hand, for oral administration of a non-fermentable disaccharide selected from lactulose and lactitol in combination with neomycin (more correctly neomycin B), generally in salified form and, preferably, in sulphate form;
- on the other hand, for parenteral administration of branched amino-acids and balanced glucose solutions.

With regard to the first stage, neomycin performs the function of inactivating the nitrogenous-toxin-producing bacteria which are in the colon, thus reducing the synthesis of ammonia. Lactulose and lactitol, on the other hand, are broken down to organic acids by the intestinal bacterial flora, reduce the pH in the colon, and inhibit the production of ammonia by the intestinal bacterial flora.

In contrast with the first stage of the treatment which is directed precisely towards blocking the synthesis of nitrogenous toxins, the second stage, on the other hand, is directed towards opposing the action, at cerebral level, of the nitrogenous toxins which are nevertheless formed; the branched amino-acids and glucose in fact penetrate the brain cells where they compete with the nitrogenous neurotoxins, reestablishing the neuron function which preceded the occurrence of the encephalopathy.

The treatment described above, however, is associated with a series of contraindications of considerable significance.

In the first place, the oral administration of any solution or suspension during the second, third or fourth stages of the disease is often very problematical or even impossible because of the patient's inability to coordinate the physiological swallowing movements.

In the second place, neomycin is an antibacterial drug which is known for its toxic side effects; these toxic effects in fact develop in the vestibular and renal sites (nephrotoxicity and ototoxicity) and are directly dose-dependent. Neomycin taken orally is normally absorbed in the gastrointestinal tract to an extent of 1-3% and eliminated in unmodified form with the faeces to an extent of about 96% (with reference to the dose taken); however, pathological conditions frequently associated with cirrhosis of the liver, such as gastric or duodenal ulcers, congestive gastropathy, or intestinal inflammation, may cause an increase in the absorption of orally-administered neomycin, thus considerably increasing the risks of intoxication. For this reason, neomycin is normally administered orally only in the vigil or declared coma stages.

Both lactulose and lactitol, on the other hand, are normally administered from the prodromic stage, in increasing daily oral doses of 40-80 g; however, although they do not have the typical toxicity of neomycin, they are nevertheless also associated with unpleasant phenomena such as, for example, nausea, vomiting, flatulence, diarrhoea and abdominal pains.

Glucose and branched amino-acids also have limitations for administration in cirrhotics in view of the considerable frequency of cases of associated diabetes and renal insufficiency (hepato-renal syndrome).

The object of the present invention is therefore to find a method of treating hepatic encephalopathy which is free of the undesirable effects and adverse reactions listed above.

It has now been found that if an aqueous solution containing neomycin and a non-fermentable disaccharide, preferably lactitol or lactulose, is administered by enteroclysis, favourable results are achieved both with regard to therapeutic effects and with regard to the elimination or at least reduction of these contraindications.

The aqueous solution which constitutes the subject of the present invention contains a quantity of disaccharide preferably of between 0.05 and 0.5 g/ml, and even more preferably, of between 0.1 and 0.3 g/ml, and a quantity of neomycin preferably of between 0.05 and 5 g/l, and even more preferably about 1-2 g/l. The disaccharide in question is preferably selected from lactitol and lactulose; with regard to neomycin (or, more correctly, neomycin B), this may be used as such or in the form of one of its salts, preferably in sulphate form.

The aqueous solutions according to the present invention may be prepared, at the time of use, by adding, directly to spring water, neomycin and the non-fermentable disaccharide, both of which are generally in crystalline form and soluble in water.

Plastics devices are generally used for the preparation, at the time of use, of the aqueous solution according to the present invention which may contain excipients, coadjuvants and/or preservatives. Particularly preferred are devices ready for use for the preparation of single-dose aqueous solutions, at the time of use, (simply by adding water at about 37°C and stirring briefly to dissolve the contents) and for subsequent administration, for example, disposable enteroclysms having a final volume of 500-1500 ml, preferably 1000 ml, containing from 100 to 300 g, preferably 200 g, of disaccharide and from 0.05 to 5 g, preferably 1-2 g, of neomycin. A device of the type in question is preferably constituted by a graduated bag, complete with an outlet and rectal cannula, acting as a container and as administration apparatus; even more preferably, the graduated bag contains the disaccharide in solid form and, for reasons of hygiene and stability, the neomycin is in a separate package (for example, a single-dose sachet). At the time of preparation, water is added to the bag containing the disaccharide until the desired volume is reached; the neomycin is added and the solution is then ready for use.

A second subject of the present invention is therefore represented by preparations in solid form for the preparation of aqueous solutions according to the invention, at the time of use.

Finally, a further subject of the present invention is represented by the use of at least one non-fermentable disaccharide in combination with neomycin for the preparation of aqueous enteroclysis solutions for the treatment of hepatic encephalopathy.

Since the oral administration of neomycin and lactulose is known, at first sight, it was not to be expected that the use of the rectal route would lead to strictly therapeutic advantages. Investigations carried out on patients suffering from hepatic encephalopathy and subjected to rectal treatment with an aqueous solution as described above have shown therapeutic results which are very significant when compared with those which can normally be achieved by conventional methods, as well as easier administration of the treatment in cases of imminent, alert or apparent coma. In particular, it has been found that both the times taken to awake from states of vigil or declared coma and the number of comatose episodes per month are reduced to a substantial extent in comparison with those normally occurring in patients subjected to conventional treatment.

It has also been found that, in the patients subjected to rectal treatment with a solution according to the present invention, the absorption of neomycin does not in any case exceed the level of 1-3%, even in patients suffering from cirrhosis of the liver, gastric or duodenal ulcers, congestive gastropathy, or intestinal inflammation, thus avoiding the risk of neomycin intoxication.

Similarly, the results obtained at the level of inhibition of the synthesis of ammonia or, in any case, of other nitrogenous toxins as a result of the treatment have been found considerably superior to those achieved by conventional treatment; in particular, it has been found that, in patients subjected to enteroclysis with an aqueous solution according to the present invention, the administration of branched amino-acids and glucose can be eliminated or, in any case, limited purely to cases of declared coma, with clear advantages for diabetic patients or, in any case, patients suffering from renal insufficiency.

Finally, it has been found that the rectal treatment in question enables neomycin and the non-fermentable disaccharide to be administered in considerably greater quantities than are permitted by the prior art; in particular, it has been noted that a treatment of this type enables as much as 200 g of lactitol to be administered in a single dose which, if administered orally, would not be tolerated by the patient.

These and further advantages of the present invention will become clear from the following examples which should be considered purely as non-limiting examples of the invention.

### Example 1

12 patients suffering from PSE, aged between 34 and 74 years and all having been treated orally, for a period of at least 6 months, with lactulose (50 g/day in the first and second stages) with the addition of neomycin (3g/day for at least 7 days) in cases of vigil or declared coma, were subjected to treatment by enteroclysis with a solution containing 200 ml of lactulose and 1g (in patients in a vigil coma state) or 2 g (in patients in a state of declared coma) of neomycin sulphate dissolved in 1000 ml of spring water which was administered every 6 hours to the patients in a state of vigil or declared coma until they were fully awakened.

Therapeutic effectiveness was evaluated principally on the basis of the reduction in awakening times which, in patients subjected to conventional treatment (oral treatment with neomycin and lactulose), were, on average, 48-72 hours; the results are given in the following table.

**Table 1**

| Patient No. | Age | Sex | Awakening time |
|---|---|---|---|
| 1 | 64 | Male | 10 hours |
| 2 | 59 | Female | 11 hours |
| 3 | 34 | Male | 12 hours |
| 4 | 56 | Female | 9 hours |
| 5 | 69 | Male | 10 hours |
| 6 | 57 | Male | 9 hours |
| 7 | 61 | Male | 8 hours |
| 8 | 74 | Female | 8 hours |
| 9 | 73 | Female | 9 hours |
| 10 | 71 | Female | 10 hours |
| 11 | 68 | Female | 8 hours |
| 12 | 70 | Female | 12 hours |

As it can be seen from the table, the results achieved by the administration of the solution by enteroclysis in accordance with the present invention are very encouraging since they permit a reduction of about 70-75% in the awakening times from stages 3 and 4 of PSE, in comparison with conventional oral treatment. After the treatment described above, it was possible to observe a substantial reduction in the number of comatose episodes per month which changed from an average number of 3-5 comatose episodes per month to maximum points of 2 episodes.

Finally, on the basis of the positive effects described, it was possible to omit the parenteral administration of glucose solutions and/or branched amino-acids.

## Claims

1. Use of at least one non fermentable disaccharide in combination with neomycin for the manufacture of an aqueous solution for enteroclysis application for the treatment of hepatic encephalopathy.

2. Use according to claim 1, **characterized in that** said non fermentable disaccharide is selected from lactulose and lactitol.

3. Use according to claim 1, **characterized in that** neomycin is present in the form of one of its salt.

4. Use according to claim 3, **characterized in that** neomycin is present in sulphate form

5. Use according to claim 1, **characterized in that** said non fermentable disaccharide is present in a quantity of between 0.05 and 0.5 g/ml of aqueous solution, preferably of between 0.1 and 0.3 g/ml.

6. Use according to claim 1, **characterized in that** neomycin is present in a quantity of between 0.05 and 5 g/l of aqueous solution, preferably of between 1 and 2 g/l.

7. Use according to claim 1, **characterized in that** the hepatic encephalopathy is of porto-systemic type.

8. Use according to claim 1, **characterized in that** said aqueous solution for enteroclysis application is prepared at the time of use.

9. Use according to claim 8, **characterized in that** said aqueous solution for enteroclysis application is a single-dose one.

10. Use according to claim 9, **characterized in that** said aqueous solution for enteroclysis contains, in addition, excipients, coadjuvants and/or preservatives.

## Patentansprüche

1. Verwendung von mindestens einem nicht fermentierbaren Disaccharid in Kombination mit Neomycin zur Herstellung einer wässrigen Lösung zur Enteroclyseanwendung bei der Behandlung der hepatischen Enzephalopathie.

2. Verwendung gemäß Anspruch 1, **dadurch gekennzeichnet, dass** das nicht fermentierbare Disaccharid aus Lactulose und Lactitol ausgewählt ist.

3. Verwendung gemäß Anspruch 1, **dadurch gekennzeichnet, dass** das Neomycin in Form eines seiner Salze vorliegt.

4. Verwendung gemäß Anspruch 3, **dadurch gekennzeichnet, dass** das Neomycin in Sulfatform vorliegt.

5. Verwendung gemäß Anspruch 1, **dadurch gekennzeichnet, dass** das nicht fermentierbare Disaccharid in einer Menge von zwischen 0,05 und 0,5 g/ml einer wässrigen Lösung, bevorzugt zwischen 0,1 und 0,3 g/ml vorliegt.

6. Verwendung gemäß Anspruch 1, **dadurch gekennzeichnet, dass** das Neomycin in einer Menge zwischen 0,05 und 5 g/l einer wässrigen Lösung, bevorzugt zwischen 1 und 2 g/l vorliegt.

7. Verwendung gemäß Anspruch 1, **dadurch gekennzeichnet, dass** die hepatische Enzephalopathie vom portalsystemischen Typ ist.

8. Verwendung gemäß Anspruch 1, **dadurch gekennzeichnet, dass** die wässrige Lösung zur Enteroclyseanwendung zum Verwendungszeitpunkt hergestellt wird.

9. Verwendung gemäß Anspruch 8, **dadurch gekennzeichnet, dass** die wässrige Lösung zur Enteroclyseanwendung eine Einzeldosis ist.

10. Verwendung gemäß Anspruch 9, **dadurch gekennzeichnet, dass** die wässrige Lösung zur Enteroclyse zusätzlich Arzneiträger, Co-Adjuvantien und/oder Konservierungsmittel enthält.

## Revendications

1. Utilisation d'au moins un disaccharide non fermentescible en association avec la néomycine dans la fabrication d'une solution aqueuse pour entéroclyse pour le traitement de l'encéphalopathie hépatique.

2. Utilisation selon la revendication 1, **caractérisée en ce que** ledit disaccharide non fermentescible est choisi parmi le lactulose et le lactitol.

3. Utilisation selon la revendication 1, **caractérisée en ce que** la néomycine est présente sous la forme d'un de ses sels.

4. Utilisation selon la revendication 3, **caractérisée en ce que** la néomycine est présente sous la forme de sulfate.

5. Utilisation selon la revendication 1, **caractérisée en ce que** ledit disaccharide non fermentescible est présent en quantité comprise entre 0,05 et 0,5 g/ml de solution aqueuse, de préférence entre 0,1 et 0,3 g/ml.

6. Utilisation selon la revendication 1, **caractérisée en ce que** la néomycine est présente en quantité comprise entre 0,05 et 5 g/l de solution aqueuse, de préférence entre 1 et 2 g/l.

7. Utilisation selon la revendication 1, **caractérisée en ce que** l'encéphalopathie hépatique est de type porto-systémique.

8. Utilisation selon la revendication 1, **caractérisée en ce que** ladite solution aqueuse pour entéroclyse est préparée au moment de son utilisation.

9. Utilisation selon la revendication 8, **caractérisée en ce que** ladite solution aqueuse pour entéroclyse est une dose unitaire.

10. Utilisation selon la revendication 9, **caractérisée en ce que** ladite solution aqueuse pour entéroclyse contient, en plus, des excipients, des coadjuvants et/ou des conservateurs.
